# EUROPEAN PATENT SPECIFICATION

(11) **EP 3 699 142 B1**
(45) Date of publication and mention of the grant of the patent: **30.09.2026**
(21) Application number: 19461514.2
(22) Date of filing: 20.02.2019
(51) Int. Cl.: C12Q 1/6834, C01B 32/184, C01B 32/194, B82Y 15/00

(54) **METHOD OF IMMOBILIZATION NUCLEIC ACID STRUCTURE ON SURFACE OF GRAPHENE, GRAPHENE MODIFIED BY IMMOBILIZATION OF NUCLEIC ACID STRUCTURE AND ITS USE**
VERFAHREN ZUR IMMOBILISIERUNG DER NUKLEINSÄURESTRUKTUR AUF DER OBERFLÄCHE VON GRAPHEN, DURCH IMMOBILISIERUNG DER NUKLEINSÄURESTRUKTUR MODIFIZIERTES GRAPHEN UND DESSEN VERWENDUNG
PROCÉDÉ D'IMMOBILISATION D'UNE STRUCTURE D'ACIDE NUCLÉIQUE SUR UNE SURFACE DE GRAPHÈNE, GRAPHÈNE MODIFIÉ PAR L'IMMOBILISATION DE LA STRUCTURE D'ACIDE NUCLÉIQUE ET SON UTILISATION

(43) Date of publication of application: 26.08.2020
(73) Proprietor: Instytut Chemii Fizycznej Polskiej Akademii Nauk, 01-224 Warszawa (PL); Ludwig-Maximilians-Universität München, 80539 München (DE)
(72) Inventor: KAMINSKA, Izabela, Gdynia (PL); TINNEFELD, Philip, 80999 München (DE)
(74) Representative: Witek, Rafal

(56) References cited:
- WO-A1-2017/112941
- GUANGFU WU ET AL: "Graphene Field-Effect Transistors for the Sensitive and Selective Detection of Escherichia coli Using Pyrene-Tagged DNA Aptamer", ADVANCED HEALTHCARE MATERIALS, vol. 6, no. 19, 1 October 2017 (2017-10-01), DE, pages 1700736, XP055584620, ISSN: 2192-2640, DOI: 10.1002/adhm.201700736
- GUANGFU WU ET AL, SUPPLEMENTARY MATERIAL: "Graphene Field-Effect Transistors for the Sensitive and Selective Detection of Escherichia coli Using Pyrene-Tagged DNA Aptamer", ADVANCED HEALTHCARE MATERIALS, vol. 6, no. 19, 1 October 2017 (2017-10-01), DE, pages 1700736, XP055584661, ISSN: 2192-2640, DOI: 10.1002/adhm.201700736
- ANASTASIYA PUCHKOVA ET AL: "DNA Origami Nanoantennas with over 5000-fold Fluorescence Enhancement and Single-Molecule Detection at 25 [mu]M", NANO LETTERS, vol. 15, no. 12, 9 December 2015 (2015-12-09), US, pages 8354 - 8359, XP055584731, ISSN: 1530-6984, DOI: 10.1021/acs.nanolett.5b04045
- CAROLIN VIETZ ET AL: "Functionalizing large nanoparticles for small gaps in dimer nanoantennas", NEW JOURNAL OF PHYSICS, INSTITUTE OF PHYSICS PUBLISHING, BRISTOL, GB, vol. 18, no. 4, 15 April 2016 (2016-04-15), pages 45012, XP020300527, ISSN: 1367-2630, [retrieved on 20160415], DOI: 10.1088/1367-2630/18/4/045012
- DATABASE WPI Week 201141, Derwent World Patents Index; AN 2011-E00622, XP002790946

## Description

The present invention relates to a method of immobilizing nucleic acid structure such as DNA Origami on a surface of graphene, the graphene modified by immobilization of nucleic acid structure and its use.

### Background of the invention

### DNA Origami

The present invention is based on "Structural DNA nanotechnology" and "Scaffolded DNA Origami" techniques. The former approach, initiated in the early 1980s by Nadrian Seeman, takes advantage of the molecular recognition properties of synthetic DNA oligonucleotides to create finite size objects or infinite periodic lattices through complementary Watson-Crick base pairing, sticky-end cohesion and stacking interactions. Scaffolded DNA Origami, introduced in 2006 by Paul Rothemund, simplifies the access to finite DNA nanoobjects. In this technique, single-stranded circular DNA molecules of typically 7 - 8.5 kb size are folded by the aid of staple strands into an array of helices through an arrangement of periodic crossovers. Extension of this concept to assemble 3D DNA structures has led to the establishment of a robust and reliable method to fabricate DNA nanostructures with dimensions in the 20 to 200 nm range. Origami structures can be used as "molecular pegboards" with an addressable surface area of a few thousand nm² for arranging arbitrary objects-of-interest (001) with a "single pixel" resolution of about 6 nanometers. Typical OOls are metal or semiconductor nanoparticles for potential applications in microelectronics and (non-linear) optics, or proteins, peptides and other ligands for potential applications in biosensing, cell culture, or other areas of biological and biomedical research. In this context, DNA origami structures could advantageously allow for control of the absolute number, stoichiometry and precise nanoscale orientation of OOls, e.g. receptors and additional cell membrane proteins for presentation to living cells. Many of the aforementioned potential applications require or would at least benefit from methods to immobilize DNA origami structures on solid supports, in particular in a site-specific manner. So far, nanoparticle-decorated origami structures have been deposited on nanopatterned surfaces prepared by electron-beam lithography and etching processes to create DNA origami shaped binding sites on non-transparent substrates. While these approaches proved feasible for generating large-area spatially ordered arrays of DNA origami, the production of substrates is very cost and time consuming. Even more important, site-selectivity could not yet be realized because origami immobilization is governed by mere electrostatic interactions.

### Graphene

Graphene is a unique two-dimensional 2D material with promising applications in the nanoscience. A main limitation has been the problem of selective and controlled chemical modification without disturbing its properties. This has limited its usefulness especially in electronic and optical sensing applications.

Graphene is a 2D carbon lattice resembling a honeycomb, which has attracted great attention since 2004, when it was experimentally isolated for the first time. Due to its unique electronic, optical and mechanical properties, it has been intensively explored worldwide and found applications probably in every branch of science (Ferrari et al., 2014). Its gapless energy band structure and linear dispersion relation near the corners of the Brillouin zone result in a frequency-independent light absorption, governed solely by the fine-structure constant, *α*≈ 1/137. As a result, this only one-atom thick material absorbs as much as πα≈2.3% of light, over the visible and near-infrared spectral regions.(Nair et al., 2008) In consequence, graphene behaves as an extraordinary energy sink and a unique acceptor system, which is one of the key characteristics of graphene and graphene-related twodimensional materials exploited in the field of optical biosensors and distance rulers (Anichini et al., 2018; Zhu, Du, & Lin, 2015). Fluorescent dyes placed close to graphene are strongly quenched and their displacement from graphene can restore fluorescence (Chen, Berciaud, Nuckolls, Heinz, & Brus, 2010; Federspiel et al., 2015; Gaudreau et al., 2013; Mazzamuto et al., 2014). It has been demonstrated both theoretically and experimentally that the energy transfer from a molecule (a single dipole) to graphene ("2D array of dipoles") strongly depends on the distance d between both, and scales proportional to *d⁻⁴* (Brenneis et al., 2015; Gaudreau et al., 2013; Swathi & Sebastian, 2008, 2009). Whereas the distance dependence is well understood, reports vary with respect to the *do* value which states the distance of 50% quenching efficiency. This variation is related to the different emitters used (e.g. quantum dots, nitrogen-vacancy centers or dyes embedded in crystals) and how the distance to the graphene layer was controlled, and reported values range from *d₀=* 8 to 20 nm (Brenneis et al., 2015; Chen et al., 2010; Federspiel et al., 2015; Gonyalves et al., 2016; Mazzamuto et al., 2014). In one work quenching up to 60 nm was reported (Gonyalves et al., 2016).

Hitherto, gold surfaces and not graphene surfaces are commonly used in fluorescence quenching biosensing as besides the quenching a well-developed surface chemistry exists. Gold has thus been the material of choice for biosensing (Langer, A. et al.; Sci. Rep. 2015, 5 (January), 1-15; Schlichtiger, A. et al.; Anal. Bioanal. Chem. 2013, 405 (1), 275-285; Langer, A. et al.; Nat. Commun. 2013, 4), as well as for MIET (Chizhik, A. I. et al.; Nat. Photonics 2014, 8 (February), 124-127; Isbaner, S. et al.; Nano Lett. 2018, 18 (4), 26162622) (metal induced energy transfer) super-resolution imaging. Compared to gold, graphene offers the outstanding advantage of good optical transparency in the far-field and less background fluorescence. So far, however, graphene and other related 2D materials lack the chemical flexibility to carry out complex biomolecular assays. Main problems include missing control over surface chemistry, construct composition and fabrication, as well as low reproducibility of graphene-based hybrid structures.

It was demonstrated with TEM imaging that DNA origami nan opiates were denatured due to hydrophobic interactions of the DNA bases with graphene upon adsorption.(Green, Pham, Crow, Burke, & Norton, 2018; Kabiri et al., 2017).

In the publication "Graphene Field-Effect Transistors for the Sensitive and Selective Detection of Escherichia coli Using Pyrene-Tagged DNA Aptamer" (GUANGFU WU et al., ADVANCED HEALTHCARE I\/IATERIALS, vol. 6, no..19,.1 October 2017 A (2017-10-01)), biosensing use of graphene field-effect transistors with the aid of pyrene-tagged DNA aptamers, which exhibit excellent selectivity, affinity, and stability for Escherichia coli (E. coli) detection was reported.

In another publication "DNA Origami Nanoantennas, with over 5000-fold Fluorescence Enhancement and Single-Molecule Detection at 25 [mu]M" (ANASTASIYA PUCHKOVA_et al., ,NANO LETTERS, vol. 15, no. 12, 9 December 2015 (2015-12-09)) new generation of self-assembled DNA origami based optical nanoantennas with improved robustness, reduced interparticle distance, and optimized quantum-yield improvement to achieve more than 5000-fold fluorescence enhancement and single-molecule detection at 25 µM background fluorophore concentration was presented.In a further publication "Functionalizing large nanoparticles for small gaps in dimer nanoantennas" (CAROLIN VIETZ et al., NEW JOURNAL OF PHYSICS, INSTITUTE OF PHYSICS PUBLISHING, BRISTOL, GB, vol. 18, no.4, 15 April 2016 (2016-04-15)) arranged with use of zipper geometry nanoparticles of 100 and 150 nm diameter on DNA origami and formed gap nanoantennas were disclosed.

An international patent application WO2017/112941A1 discloses methods, systems, and nano-sensor devices for detecting or discriminating nucleic acids with a single nucleotide resolution based on nucleic acid strand displacement.

The pyrene-modified oligonucleotide (staple strands) are known from the publication "Recent Advances in Nucleic Acid Targeting Probes and Supramolecular Constructs Based on Pyrene-Modified Oligonucleotides" (Olga A. Krasheninina et al.; Molecules 2017, 22, 2108*).* The exact sequence of it depends only on the sequence in the DNA origami structure, since it is incorporated *via* hybridization with complementary protruding strands. It is therefore an external modification not influencing the structure itself.

Accordingly, the technical problem underlying the present invention is how to have a well defined chemical modification of graphene to make its quenching properties exploitable for biochemical sensing assays. The solution is to use nucleic acid structures such as DNA origami as chemical adapters. Beyond the mere chemical adaptation of any assay that might be incorporated within the DNA origami, the DNA origami is also a nanopositioner: it can place the assay at the height where the fluorescence signal is most sensitive to minimal distance alterations.

Another technical problem underlying the present invention is to provide methods for immobilization of nucleic acid structures such as DNA origami structures on graphene. Further, respective assemblies of immobilized nucleic acid structures such as DNA origami on graphene and uses thereof should be provided.

The solution to the above technical problems is achieved by the embodiments characterized in the claims.

### Summary of the invention

The subject of the invention is a method of immobilization of nucleic acid structures on graphene, said method comprising the steps of:
i) providing at least one type of DNA origami structures having at least one type of a molecule-modified oligonucleotide, wherein said molecule is able to attach to graphene via π-interactions, wherein said molecule which is able to attach to graphene via π-interactions is pyrene,
ii) the DNA origami structures having at least one type of the molecule-modified oligonucleotide are contacted with a graphene layer at a temperature from the range of 10-40°C, to immobilize the DNA origami structures on graphene.

Preferably, step i) comprises the following sub-steps of:
- providing at least one type of a DNA origami structure having at least one type of an oligonucleotide,
- providing at least one type of a molecule-modified oligonucleotide, wherein said molecule is able to attach to graphene via π-interactions, wherein said molecule which is able to attach to graphene via π-interactions is pyrene, and wherein the molecule-modified oligonucleotide comprising nucleobases complementary to purines or pyrimidines is complementary in the Watson-Crick meaning to at least one type of the oligonucleotide comprised in the DNA origami structure, wherein the complementary oligonucleotide comprises nucleobases selected from purines or pyrimidines,
- the DNA origami structure with the molecule-modified oligonucleotide is incubated for more than 5 min, preferably more than 30 min, more preferably for 30-120 min, at a temperature from the range of 10-40°C, more preferably 30-38°C, to pair in the Watson-Crick manner between the oligonucleotides of the DNA origami structures and the complementary molecule-modified oligonucleotide.

Preferably, step i) comprises the following sub-step of forming DNA origami structures by use of molecule-modified staple strands, wherein said molecule is able to attach to graphene via π-interactions.

Preferably, step ii) is carried out for at least 10 seconds, and/or step ii) is carried out at temperature from the range of 15-30°C.

Preferably, said method comprising the steps of:
a) providing at least one type of a DNA origami structure having at least one type of an oligonucleotide protruding from the DNA origami structure and/or built into the DNA origami structure,
b) providing at least one type of a molecule-modified oligonucleotide, wherein said molecule able to attach to graphene via π-interactions is pyrene, and wherein said oligonucleotide comprising nucleobases complementary to purines or pyrimidines is complementary in the Watson-Crick meaning to the oligonucleotides protruding from the DNA origami structure and/or built into the DNA origami structure, wherein the complementary oligonucleotide comprises nucleobases selected from purines or pyrimidines,
c) the DNA origami structure with the molecule-modified oligonucleotide is incubated for more than 5 min, preferably more than 30 min, more preferably for 30-120 min, at a temperature from the range of 10-40°C, more preferably 30-38°C, to pair in the Watson-Crick manner between the oligonucleotides protruding from the DNA origami structure and/or built into the DNA origami structure and the complementary molecule-modified oligonucleotides,
d) the incubated DNA origami structure is contacted with a graphene layer for more than 10 seconds, at a temperature from the range of 10-40°C, to immobilize the DNA origami structures on the graphene layer.

Preferably, said molecule able to attach to graphene via TT-interactions is pyrene, and/or the number of pyrene molecules per one DNA origami structure is at least 2, preferably 4-10.

Preferably, incubation in step c) is carried out for 2 hours in 37°C.

Preferably, said the DNA origami structure structures carry one or more type of objects-of-interest (OOI), wherein the OOls are selected from the group comprising proteins, peptides, nanoparticles, carbohydrates, small molecule ligands, dyes, nanocrystals, DNA, RNA, chemical moieties such as thiol or biotin.

Preferably, after step of immobilization of the DNA origami structure on the graphene layer, a further step of washing the immobilized sample with any solution in which DNA origami is stable, such as ultrapure water, Phosphate Buffered Saline buffer, 10 mM Tris or 1xTris-EDTA buffer containing 1-15 mM MgCl₂, is carried out..

Another subject of the present invention is graphene modified by immobilization of DNA origami structures and a graphene layer, wherein the DNA origami structures are immobilized on the graphene layer via molecule-modified oligonucleotides, wherein said molecule which is able to attach to graphene via π-interactions is pyrene, wherein said molecule is able to attach to graphene via π-interactions, and wherein said molecule-modified oligonucleotides comprising nucleobases complementary to purines or pyrimidines are complementary in the Watson-Crick meaning for pairing to the oligonucleotides comprised in said nucleic acid structures, wherein the complementary oligonucleotides comprise nucleobases selected from purines or pyrimidines.

Another subject of the present invention is graphene modified by immobilization of DNA origami structure is immobilized on the graphene layer via pyrene-modified oligonucleotides comprising nucleobases complementary to purines or pyrimidines, complementary in the Watson-Crick meaning for pairing to the oligonucleotides protruding from the DNA origami structure and/or built into said DNA origami structure, wherein the complementary oligonucleotides comprise nucleobases selected from purines or pyrimidines..

Preferably, said graphene modified by immobilization of DNA origami structures according to claims 11 or 12, wherein DNA origami structures carry one or more types of objects-of-interest (OOI), wherein the OOls are selected from the group comprising proteins, peptides, nanoparticles, carbohydrates, small molecule ligands, dyes, nanocrystals, DNA, RNA, chemical moieties such as thiol or biotin..

Another subject of the present invention is use of graphene modified by immobilization of DNA origami structures defined in any of claims 10-12 in biosensing and energy conversion as well as photovoltaics and optoelectronics.

In particular, the above technical problem is overcome by using DNA origami surface chemistry converters/nanopositioners. DNA origamis are programmed nanostructures of roughly 21.000 nm³. Here, by incorporating pyrene moieties on specific DNA strands it is possible to selectively place DNA origami nanostructures on top of graphene. As DNA origami nanostructures can be functionalized in many other ways the surface chemistry can be adapted from a graphene surface to many other chemical functionalities.

Pyrene (Benzo[def]phenanthrene, CAS Number 129-00-0) is a polycyclic aromatic hydrocarbon (PAH) consisting of four fused benzene rings, resulting in a flat aromatic system. The chemical formula is C16H10. This colorless solid is the smallest peri-fused PAH (one where the rings are fused through more than one face). Pyrene forms during incomplete combustion of organic compounds.

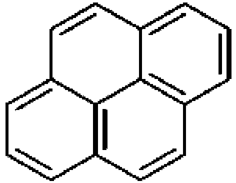

The usefulness of DNA origami surface chemistry converters by placing single dye molecules at specific, designed distances in the range of 1-60 nm from the graphene surface is demonstrated. With this arrangement the distance dependent fluorescence quenching of dyes that will be exploited for biosensing assays in the future is determined. Besides changing surface chemistry, the fluorescence assay can be placed at a distance to the graphene surface that exhibits highest sensitivity to fluorescence changes.

### Brief description of the figures

Figure 1. Sketches of rectangular-, disc- and pillar-shaped DNA origami structures. A single ATT0542 fluorophore (green sphere) is positioned at a height of 3 nm *(NR),* 7 nm *(ND),* 12 nm *(NP1),* 16 nm *(NP2) ,* 24 nm *(NP3)* and 53 nm *(NP4).* A blue frame: zoom-in of pyrene- modified (orange) DNA strand protruding from DNA origami and interacting with graphene via π- π interactions. Grey bars represent double-stranded DNA.
Figure 2. Fluorescence intensity images (5×5 µm) obtained for DNA origami structures with a single dye (ATT0542) immobilized on graphene, for excitation wavelength 532 nm, and laser power ranging from 1 to 5 µW *(NR* - 5 µW, *ND* and *NP1-* 2 µW, *NP2, NP3* and *NP4 - 1* µW).
Figure 3. Examples of fluorescence transients obtained for DNA origami structures with a single dye (ATT0542) immobilized on graphene, for excitation wavelength 532 nm, and laser power ranging from 1 to 5 µW *(NR* - 5 µW, *ND* and *NP1 -* 2 µW, *NP2, NP3* and *NP4 - 1* µW).
Figure 4. Raman spectrum of a single graphene layer, averaged from a map of 30 measured spots.
Figure 5. (A) Normalized fluorescence intensity decays of ATT0542 at difference distances to graphene (averaged from 20 decays for each sample). (B) Relative fluorescence intensity as a function of fluorescence lifetime (fluorescence lifetimes were obtained by reconvolution).
Figure 6. Normalized fluorescence intensity decays of Att0542 within DNA origami structure NP4 (a dye placed at the height of 53 nm) immobilized on glass (▪) or graphene (•).

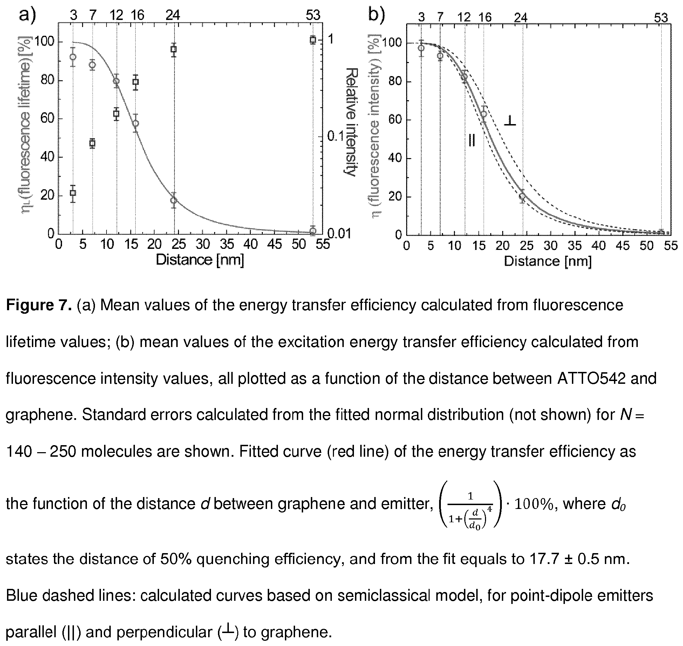

Figure 7. (a) Mean values of the energy transfer efficiency calculated from fluorescence lifetime values; (b) mean values of the excitation energy transfer efficiency calculated from fluorescence intensity values, all plotted as a function of the distance between ATT0542 and graphene. Standard errors calculated from the fitted normal distribution (not shown) for N *=* 140 - 250 molecules are shown. Fitted curve (red line) of the energy transfer efficiency as the function of the distance d between graphene and emitter, $\left(\frac{1}{1 + {\left(\frac{d}{{d}_{0}}\right)}^{4}}\right) ⋅ 100 %$ where *d₀*_states the distance of 50% quenching efficiency, and from the fit equals to 17.7 ± 0.5 nm. Blue dashed lines: calculated curves based on semiclassical model, for point-dipole emitters parallel (II) and perpendicular (⊥) to graphene.
Figure 8. (Top) TEM images of: (from left) Nanodisc and Nanopillar. (Bottom) AFM images of: (from left) Nanorectangle, Nanodisc and Nanopillar.

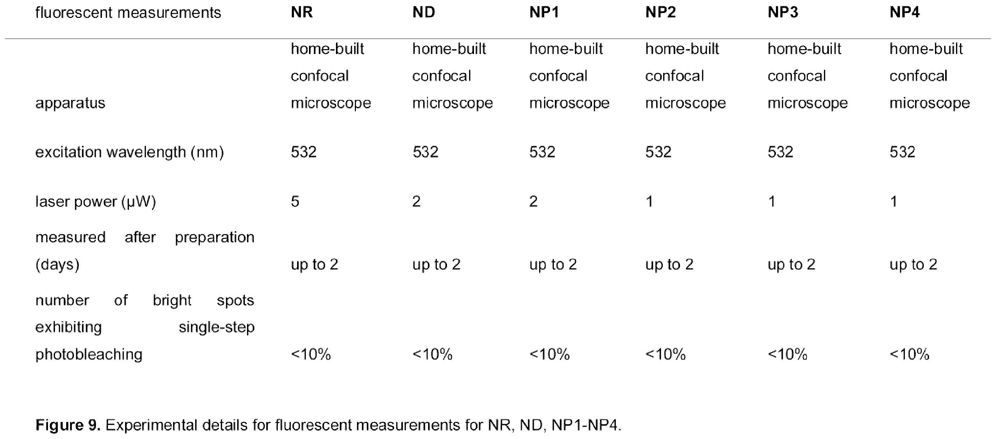

Figure 9. Experimental details for fluorescent measurements for NR, ND, NP1-NP4.

### Detailed description of the invention

The present Inventors used DNA origami (Rothemund, P. W. K. Nature 2006, 440 (7082), 297-302) as a surface chemistry converter to generally overcome the problem of chemical functionalization of graphene for optical biosensing assays. The DNA origami technique enables the formation of custom-designed DNA nanostructures with a volume of -21.000 nm³, and the rich DNA chemistry available allows placing of arbitrary objects and complex biomolecular assays (Acuna, G. P. et al.; ACS Nano 2012, 6 (4),3189-3195; Vietz, C. et al.; Nano Lett. 2017, 17, 6496-6500; Ochmann, S. E. et al.; Anal. Chem. 2017, 89, 1300013007) at programmed positions on the DNA nanostructure (Douglas, S. M. et al.; Nature 2009,459(7245),414-418; Hong, F. et al.; Chern. Rev. 2017,117,12584-12640; Ke, Y. et al.; Science (80) 2008, 319 (2008), 180-183). For biomolecular assays DNA origami can act as a biocompatible surface (Gietl, A. et al.; Nucleic Acids Res. 2012, 40 (14), 1-10). In previous works, DNA origami structures were coupled to pristine graphene either to increase their stability (Matkovic, A. et al.; New J. Phys. 2016, 18 (2), 025016) or as a template for metallized DNA nanolithography (Jin, Z. et al.; Nat. Commun. 2013, 4, 1663). On the other hand, it was demonstrated with TEM imaging that DNA origami nanoplates were denatured due to hydrophobic interactions of the DNA bases with graphene upon adsorption (Kabiri, Y. et al.; Small 2017, 1-8; Green, N. S. et al.; Mater. Res. Express 2018, 5, 045035). As a universal glue to connect the DNA origami constructs to the graphene layer without denaturation, several pyrene modified DNA strands were used that are hybridized to the DNA origami on one hand and interact with the graphene lattice via π- π stacking interactions on the other hand. It is shown that this immobilization scheme provides stable DNA origami structures for different geometries and enables placing of single, freely rotating fluorescent molecules at defined distances to the graphene layer. The exquisite distance control of single fluorescent dyes to graphene was exploited in order to revisit the distance dependence of fluorescence quenching by graphene. The narrow intensity-fluorescence lifetime distributions confirm the *cf4* law with a precise value of *d₀* of 17.7 nm in aqueous buffer solution and present the basis for a broad range of applications in biosensing and optoelectronics.

### EXAMPLES

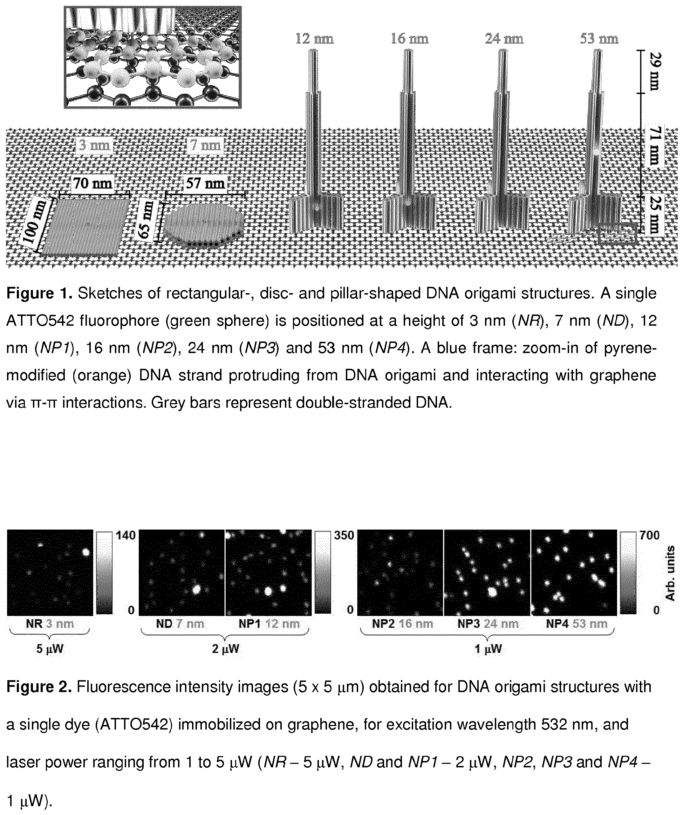

The selection of DNA origami shapes was guided by the possibility to position a single dye molecule at a designed height from the bottom (distance from graphene) and included rectangular- (nanorectangle =NR), disc- (nanodisc = *ND)* and pillar-shaped (nanopillar = *NP)* self-assembled DNA origami structures (see Figure 1). Each DNA origami structure contains one dye molecule (ATT0542) marked as a small green sphere, which is positioned at the height of: 3 nm *(NR),* 7 nm *(ND),* 12 nm *(NP1),* 16 nm *(NP2),* 24 nm *(NP3)* and 53 nm *(NP4).* The selected values cover the range of distances for which the energy transfer from a single dye molecule to graphene is expected to vary from 0 to almost 100%. As the dyes are attached to the DNA origami by 6-carbon linkers and measurements were carried out in buffer solution, it is expected that the fluorescent dyes assume all possible dipole orientations during the measurement.

Additionally, at the bottom of each structure, 6-8 staple strands with single-stranded extensions protruding from the DNA origamis were incorporated. The protrusions are used to label the DNA origami with pyrene-modified complementary oligonucleotides. The pyrene enabled selective binding of the DNA origami with the bottom side to the graphene layer (Figure 1). The stability of this interaction in certain temperature ranges (e.g. 10-40°C) is achieved by binding minimally 2 and optimally 4-10 pyrene units per one DNA origami structure, beacuse most likely one pyrene molecule is not enough to obtain stable bonds between the DNA origami and the graphene layer. External labeling with extended staple strands allowed modular modifications also for other moieties. For example, in order to measure the same samples on glass, biotin-modified oligonucleotides were used and DNA origami structures were immobilized on neutravidin-biotinylated BSA surfaces. Single-molecule fluorescence measurements were carried out with a home-built confocal microscope. Figure 2 depicts ATT0542 fluorescence intensity images for each of the six DNA origami structures using 532 nm excitation. The closer the emitter is to the graphene layer, the more its fluorescence is quenched. Therefore, we adjusted the excitation power to always work with a reasonable count rate in a regime that showed linear excitation intensity dependent emission. Laser powers ranged from 1 µW for three nanopillar samples *(NP2, NP3* and *NP4) ,* 2 µW for one nanopillar sample and the nanodisc *(NP1* and *ND),* up to 5 µW for the nanorectangle *(NR)* DNA origami structure. On all measured images, we observed that the fluorescence intensities of most spots, representing dye molecules, are very homogeneous, with occasional very bright spots.

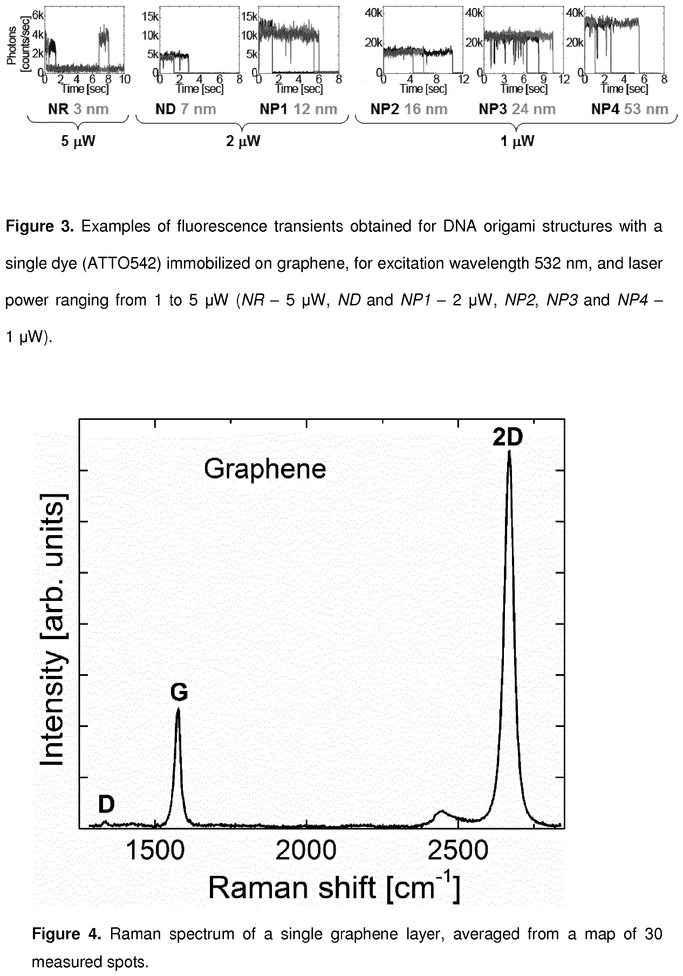

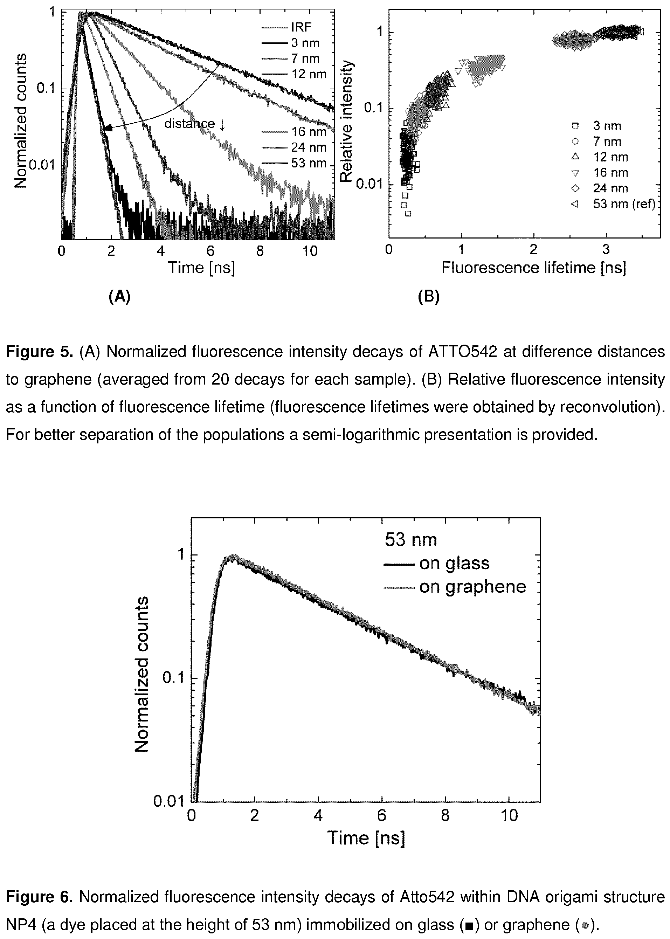

In order to characterize interactions between dye molecules and graphene, fluorescence transients were recorded for each spot. This enabled the identification of single DNA origami structures by observing blinking events and single-step photobleaching (examples of fluorescence transients are depicted in Figure 3). We identified two types of deviations from the typical single-molecule behavior, which is related to the mentioned brighter spots in the images. In cases with multi-step photobleaching, we attributed the signal to aggregates of multiple DNA origami structures. In other cases, the brighter spots exhibited single-step photobleaching and showed similar intensities and fluorescence lifetimes as molecules of the reference structures measured on glass cover slips. We hence attribute these molecules to DNA origami structures immobilized within small defects/cracks of the graphene layer. Nevertheless, taking into account the small number of such spots «10%), together with the quality control performed by Graphenea^{®} and our additional characterization using Raman spectroscopy (several tens of spectra obtained from Raman measurements, an exemplary spectrum presented in Figure 4), we conclude that graphene samples used in our measurements were of high quality. For further analysis, only transients with single bleaching steps were considered and used to determine the fluorescence intensity and fluorescence lifetime of each spot (see Imaging and Analysis for details). As expected, dye molecules incorporated in DNA origami structures bound to graphene exhibit shorter fluorescence lifetimes compared to samples immobilized on glass (see Figure 5). Only for the largest distance of 53 nm to graphene (NP4), the fluorescence properties are not affected (see Figure 6) and the unperturbed fluorescence lifetime of 3.25 ns for ATT0542 is obtained.

The reduction of fluorescence intensity and fluorescence lifetime is correlated and arise from the strong near-field interactions between the emitter and graphene. Figure 7B shows the relative fluorescence intensity $\frac{{I}_{G}}{\left〈{I}_{ref}\right〉}$ as a function of the fluorescence lifetime, where *I_{G}* is fluorescence intensity of a dye molecule within DNA origami bound to graphene, and <*I_{ref}*> is the average fluorescence intensity obtained for the reference sample, NP4. Due to the differences in the applied laser powers in our measurements, all the obtained values of fluorescence intensities were normalized to the laser power of 1 µW. It is noteworthy that narrow and clearly separated populations of fluorescence intensity and fluorescence lifetime are obtained, indicating the selectivity and robustness of the immobilization strategy. The homogeneity of the data is also fostered by the binding strategy of the dye, which can rotate during the measurement. It is therefore justified to assume that the measured data reflect the interaction of an averaged dipole orientation with the graphene layer. Interestingly, the positioning with the pyrene subunits as selective glue even yields narrow distributions for the nanopillar samples *NP1* to *NP4,* which is remarkable in view of the high aspect ratio of this DNA origami. As measurements were carried out for up to two days after sample preparation, DNA origamis on graphene are also stable and no degradation was observed.

For a quantitative analysis of the interaction between single dye molecules and graphene, and its strong distance dependence, we investigated how the quenching (relative intensity) and the energy transfer efficiency to graphene both depend on the emitter-graphene distance. The energy transfer efficiency *η* was calculated from fluorescence lifetimes $\left({η}_{L}=1-\frac{{τ}_{G}}{\left({τ}_{ref}\right)}\right)$ where *τ_{G}* is the fluorescence lifetime of a dye molecule within DNA origami bound to graphene, and *<τ_{ref}*> is the average fluorescence lifetime obtained for the reference sample, as well as from fluorescence intensities $\left({η}_{L}=1-\frac{{τ}_{G}}{\left({τ}_{ref}\right)}\right)$. Figures 4a and 4b show the distance dependent energy transfer efficiency calculated by both methods. While the shape of the graphs is similar lower energy transfer efficiencies were obtained from the fluorescence lifetime graph for the shortest distances (Figure 4a), which we attribute to uncertainty induced by the limited time resolution of the setup (see decays and instrument response function (IRF) in Figure 3a). We assume, however, that fluorescence intensity and lifetime are in this case proportional. The energy transfer efficiency obtained from intensities reaches up to 97% quenching for the smallest distance. We fitted the experimental data (Figure 4b) with the expected *d⁻⁴* dependence (red line) and obtained a *do-value* (the distance of 50% energy transfer efficiency) of 17.7 ± 0.5 nm. Additionally, in Figure 4b we compare experimental data with results calculated from the semiclassical model, which describes the near-field interactions between an emitter and graphene $\left(\frac{\left〈{τ}_{ref}\right〉}{{τ}_{G}}=1+\frac{9 να}{256 {π}^{3} {\left(ϵ+1\right)}^{2}}{\left(\frac{{λ}_{0}}{d}\right)}^{4}\right)$. In this approximation, the emitters (energy donors) are considered as classical dipoles (placed in vacuum) coupled to neighboring semi-infinite media (graphene), which acts as energy acceptor. In the equation *λ₀* states the emission wavelength (562 nm, peak emission of ATT0542) and *ε* is the permittivity of the glass substrate (2.25). In Figure 4b, we show how the intensity of the emitter decreases with distance from graphene when the dipole is oriented either parallel (II v = 1) or perpendicular (⊥= 2) to graphene (blue dashed lines). We obtained *d₀* equals 16.8 nm for v = 1 and 20.0 nm for v = 2 which is in excellent agreement with our measurement. As it is expected that the fluorescent dye is free to rotate on the flexible linker during the excited state lifetime simple geometric averaging is not meaningful but the distance dependence should be more similar to the parallel case as is also reflected in our experimental result for do.

### Example 1

### Method of Preparation

Samples of single layer CVD (chemical vapor deposition) graphene on glass coverslips were purchased from Graphenea^{®}. Quality of graphene layers were characterized using Raman spectroscopy (Figure 4).

DNA origami Nanorectangles, Nanodiscs and Nanopillars were prepared as described elsewhere (Puchkova, A. et al.; Nano Lett. 2015, 15 (12),8354-8359; Vietz, C. et al.; New J. Phys. 2016, 18,045012).

The DNA origami structures were incubated with pyrene-modified (for graphene samples) or biotin-modified (for glass samples) staple strands, complementary to the oligonucleotides protruding from DNA origami structures, for 2 h in 37°C. Such prepared structures were immobilized on the glass surface of a Lab-Tek chamber (Thermo Fisher Scientific) coated with BSA-biotin/neutravidin (Sigma-Aldrich) or directly on a single layer of CVD graphene. Finally, the sample was washed with 1×TE buffer (10 mM Tris (tris(hydroxymethyl)aminomethane), brought to pH 8.0 with HCl and 1 mM EDTA (ethylenediaminetetraacetic acid) containing 12 mM MgCb and single-molecule fluorescence measurements were performed.

The designed height values of 3 nm *(NR),* 7 nm *(ND),* 12 nm *(NP1),* 16 nm *(NP2),* 24 nm *(NP3)* and 53 nm *(NP4)* were calculated using the size parameter of a double helix, namely its diameter of 2.2 nm and a length of 0.34 nm per base pair. Taking into account previous report about the overestimation of the calculated values (the measured values smaller of about 10% compared to the designed values), these numbers were corrected. Additionally, a thickness of 1 nm for the pyrene-modified protruding strands was added.

### Example 2

### Imaging and Analysis

Single-molecule fluorescence measurements were performed on a custom built confocal microscope based on an Olympus IX71 inverted microscope. The green laser beams (532 nm LDH-P-FA-530B, Picoquant) is controlled by a PDL 828 "Sepiall" (Picoquant). The green fiber laser is decoupled by a collimator (F2220APC-532, Thorlabs). After passing through cleanup filters (532/2) and a dichroic mirror (640DCXR, AHF) the laser beam is coupled into a fiber (P3-488PM-FC, Thorlabs) with a collimator (PAF2-2A, Thorlabs) and decoupled with a collimator (G169015000, Qioptics). A combination of a linear polarizer (WP12L-Vis, Thorlabs) and a quarter wave plate (AQWP05M-600, Thorlabs) is used to obtain circularly polarized light. After passing a dual band dichroic beam splitter (z532/633, AHF), the light beam is focused by an oil-immersion objective (UPLSAPO 100XO, NA 1.40, Olympus) on the measurement chamber, which can be positioned accurately by a piezostage (P-527.3CD, PhysikInstrumente) which is driven by a E-727 controller (Physiklnstrumente). The emission of the fluorophores is collected by the same objective, focused on a 50 µm pinhole (Linos), collimated with a lens (AC050-150-A-ML, Thorlabs) and split spectrally by another dichroic beam splitter (640DCXR, AHF). The green laser beams cleaned with a filter set (HC582/75, AHF and LP 532, both Semrock) and focused with a lens (AC080-020-A-ML, Thorlabs) on the APD (SPCM-AQRH-TR-14, Excelitas) The signals of the APD are detected by a HydraHarp 400 (Picoquant) and the whole system is operated with SymPhoTime 64 (PicoQuant).

### AFM and TEM analysis of NR, ND and NP1-NP4

The correct folding of the DNA origami structures was characterized with atomic force microscopy (AFM, Nanowizard 3 ultra, JPK Instruments) in solution. On a freshly cleaved mica surface (Qualty V1, Plano GmbH) 10 µL of a 10 mM NiCl₂ x 6 H₂0 solution were incubated for 5 min. After three times washing with 300 µL miliQ-water (Merck Milli-Q) and drying with compressed air, 10 µL 1 nM DNA origami structure solution (diluted in AFM buffer (40 mM TRIS, 2 mM EDTA disodium salt dihydrate and 12.5 mM Mg(OAch x 6 H₂0) were added and incubated for 5 min. Afterwards 300 µL AFM buffer were added after purging three times with 300 µL AFM buffer. The solution measurements were performed with cantilevers USC-FO.3-kO.3-10 from Nano World.

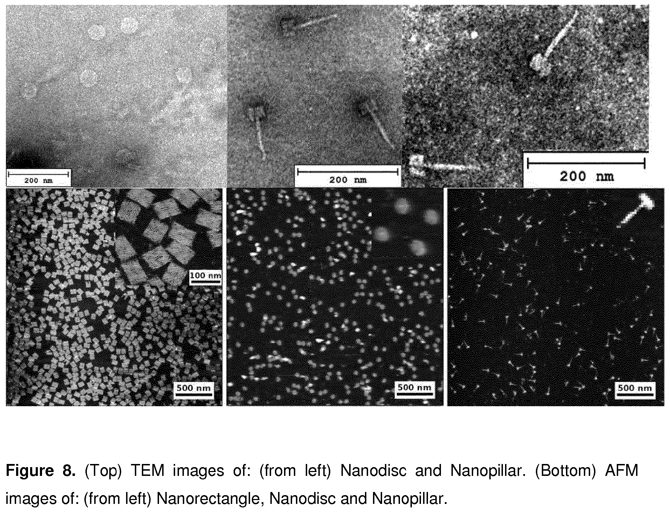

TEM grids (Ted Pella, formvar/carbon, 400 mesh, Cu) were Ar-plasma cleaned and incubated for 60 sec with DNA origami sample (5 µL, -2-10 nM). Grids were washed with 2 % uranyl formate solution (5 µL) and incubated again afterwards again 4 s with 2 % uranyl formate solution (5 µL) for staining. TEM imaging were performed with a JOEL JEM-11 00 with an acceleration voltage of 80 kV.Images of NR, ND and NP1-NP4 are given in Figure 8.

Fluorescent images are given in Figure 2.

### Conclusions

We immobilized DNA origami structures on graphene using pyrene modifications of DNA oligonucleotides. The specificity and robustness of the immobilization without denaturation enabled placing of fluorescent dyes at defined distances to the graphene layer. We confirmed the *cf4* dependence of energy transfer from the dye to graphene and determined the distance of 50% energy transfer: do = 17.7 nm. The homogeneity of the population indicates that distances to graphene can be determined with very high precision in a range of 5 - 30 nm. Together with its good transparency in the far-field graphene might become the substrate of choice for superresolution microscopy involving fluorescence lifetime measurement for determining the z-position of dyes. Beyond that, DNA origami as chemical converters will enable placing biomolecular assays on the graphene with the optimized distance to the surface for optimized sensitivity. Placing assays at a height of 17 - 18 nm will yield an extremely sensitive method of detecting small distance changes to the surface while avoiding direct contact to the interfering hydrophobic graphene surface. DNA origami as a chemical converting and placement platform could be used to incorporate further functionalities for electronic, nanophotonic, and energy conversion devices with graphene and other 2D materials opening a myriad of new possibilities.

## Claims

1. Method of immobilization of nucleic acid structures on graphene, said method comprising the steps of:
i) providing at least one type of DNA origami structures having at least one type of a molecule-modified oligonucleotide, wherein said molecule is able to attach to graphene via π-interactions, wherein said molecule which is able to attach to graphene via π-interactions is pyrene,
ii) the DNA origami structures having at least one type of the molecule-modified oligonucleotide are contacted with a graphene layer at a temperature from the range of 10-40°C, to immobilize the DNA origami structures on graphene.

2. Method according to claim 1, wherein step i) comprises the following sub-steps of:
- providing at least one type of a DNA origami structure having at least one type of an oligonucleotide,
- providing at least one type of a molecule-modified oligonucleotide, wherein said molecule is able to attach to graphene via π-interactions, wherein said molecule which is able to attach to graphene via π-interactions is pyrene, and wherein the molecule-modified oligonucleotide comprising nucleobases complementary to purines or pyrimidines is complementary in the Watson-Crick meaning to at least one type of the oligonucleotide comprised in the DNA origami structure, wherein the complementary oligonucleotide comprises nucleobases selected from purines or pyrimidines,
- the DNA origami structure with the molecule-modified oligonucleotide is incubated for more than 5 min, preferably more than 30 min, more preferably for 30-120 min, at a temperature from the range of 10-40°C, more preferably 30-38°C, to pair in the Watson-Crick manner between the oligonucleotides of the DNA origami structures and the complementary molecule-modified oligonucleotide.

3. Method according to claim 1 or 2, wherein step i) comprises the following sub-step of:
- forming DNA origami structures by use of molecule-modified staple strands, wherein said molecule is able to attach to graphene via π-interactions.

4. Method according to any of claims 1-3, wherein step ii) is carried out for at least 10 seconds, and/or step ii) is carried out at a temperature from the range of 15-30°C.

5. Method according to any of claims 1-4, said method comprising the steps of:
a) providing at least one type of a DNA origami structure having at least one type of an oligonucleotide protruding from the DNA origami structure and/or built into the DNA origami structure,
b) providing at least one type of a molecule-modified oligonucleotide, wherein said molecule able to attach to graphene via π-interactions is pyrene, and wherein said oligonucleotide comprising nucleobases complementary to purines or pyrimidines is complementary in the Watson-Crick meaning to the oligonucleotides protruding from the DNA origami structure and/or built into the DNA origami structure, wherein the complementary oligonucleotide comprises nucleobases selected from purines or pyrimidines,
c) the DNA origami structure with the molecule-modified oligonucleotide is incubated for more than 5 min, preferably more than 30 min, more preferably for 30-120 min, at a temperature from the range of 10-40°C, more preferably 30-38°C, to pair in the Watson-Crick manner between the oligonucleotides protruding from the DNA origami structure and/or built into the DNA origami structure and the complementary molecule-modified oligonucleotides,
d) the incubated DNA origami structure is contacted with a graphene layer for more than 10 seconds, at a temperature from the range of 10-40°C, to immobilize the DNA origami structures on the graphene layer.

6. Method according to claim 5, wherein said molecule able to attach to graphene via π-interactions is pyrene, and/or the number of pyrene molecules per one DNA origami structure is at least 2, preferably 4-10.

7. Method according to claim 5 or 6, wherein the incubation in step c) is carried out for 2 hours at 37°C.

8. Method according to any of claims 1-7, wherein said DNA origami structures carry one or more types of objects-of-interest (OOI), wherein the OOls are selected from the group comprising proteins, peptides, nanoparticles, carbohydrates, small molecule ligands, dyes, nanocrystals, DNA, RNA, chemical moieties such as thiol or biotin.

9. Method according to any of claims 1-8, wherein after the step of immobilization of the DNA origami structure on the graphene layer, a further step of washing the immobilized sample with any solution in which DNA origami is stable, such as ultrapure water, Phosphate Buffered Saline buffer, 10 mM Tris or 1xTris-EDTA buffer containing 1-15 mM MgCl₂, is carried out.

10. Graphene modified by immobilization of DNA origami structures and a graphene layer, wherein the DNA origami structures are immobilized on the graphene layer via molecule-modified oligonucleotides, wherein said molecule which is able to attach to graphene via π-interactions is pyrene, wherein said molecule is able to attach to graphene via π-interactions, and wherein said molecule-modified oligonucleotides comprising nucleobases complementary to purines or pyrimidines are complementary in the Watson-Crick meaning for pairing to the oligonucleotides comprised in said nucleic acid structures, wherein the complementary oligonucleotides comprise nucleobases selected from purines or pyrimidines.

11. Graphene modified by immobilization of DNA origami structures according to claim 10, where said DNA origami structure is immobilized on the graphene layer via pyrene-modified oligonucleotides comprising nucleobases complementary to purines or pyrimidines, complementary in the Watson-Crick meaning for pairing to the oligonucleotides protruding from the DNA origami structure and/or built into said DNA origami structure, wherein the complementary oligonucleotides comprise nucleobases selected from purines or pyrimidines.

12. Graphene modified by immobilization of DNA origami structures according to claim 10 or 11, wherein said DNA origami structures carry one or more types of objects-of-interest (OOI), wherein the OOls are selected from the group comprising proteins, peptides, nanoparticles, carbohydrates, small molecule ligands, dyes, nanocrystals, DNA, RNA, chemical moieties such as thiol or biotin.

13. Use of graphene modified by immobilization of DNA origami structures as defined in any of claims 10-12 in biosensing and energy conversion, as well as photovoltaics and optoelectronics.

## Patentansprüche

1. Verfahren zur Immobilisierung von Nukleinsäurestrukturen an Graphen, wobei das Verfahren die folgenden Schritte umfasst:
i) Bereitstellen von mindestens einer Art von DNA-Origami-Strukturen, die mindestens eine Art von Oligonukleotiden mit modifizierten Molekülen aufweist, wobei dieses Molekül fähig ist, durch π-Wechselwirkungen an Graphen zu binden, wobei dieses Molekül, das durch π-Wechselwirkungen an Graphen binden kann, Pyren ist.
ii) wobei die DNA-Origami-Strukturen, die mindestens eine Art von Oligonukleotiden mit modifizierten Molekülen aufweisen, in einer Temperatur von 10-40°C mit einer Graphenschicht kontaktiert werden, um die DNA-Origami-Strukturen am Graphen zu immobilisieren.

2. Verfahren nach Anspruch 1, wobei der Schritt i) die folgenden Teilschritte umfasst:
- Bereitstellen von mindestens einer Art von DNA-Origami-Strukturen, die mindestens eine Art von Oligonukleotiden aufweist,
- Bereitstellen von mindestens einer Art von Oligonukleotiden mit modifizierten Molekülen, wobei dieses Molekül fähig ist, durch π-Wechselwirkungen an Graphen zu binden, wobei dieses Molekül, das durch π-Wechselwirkungen an Graphen binden kann, Pyren ist, und wobei das Oligonukleotid mit modifizierten Molekülen, das zu Purinen oder Pyrimidinen komplementäre Nukleobasen aufweist, zu mindestens einem Typ des in der DNA-Origami-Struktur enthaltenen Oligonukleotids im Watson-Crickschen Sinne komplementär ist, wobei das komplementäre Oligonukleotid Nukleobasen enthält, die aus Purinen oder Pyrimidinen ausgewählt sind,
- die DNA-Origami-Struktur mit dem Oligonukleotid mit modifizierten Molekülen wird länger als 5 min inkubiert, bevorzugt länger als 30 min, bevorzugter 30-120 min, bei einer Temperatur im Bereich von 10-40 °C, bevorzugt 30-38°C, um eine Watson-Crick-Paarung zwischen den Oligonukleotiden der DNA-Origami-Strukturen und dem komplementären Oligonukleotid mit modifizierten Molekülen herzustellen.

3. Verfahren nach Anspruch 1 oder 2, wobei der Schritt i) die folgenden Teilschritte umfasst:
- Bildung von DNA-Origami-Strukturen durch Verwendung von molekülmodifizierten Heft-Strängen mit modifizierten Molekülen, wobei das Molekül über π-Wechselwirkungen an Graphen binden kann.

4. Verfahren nach einem der Ansprüche 1 bis 3, wobei Schritt ii) mindestens 10 Sekunden lang durchgeführt wird und/oder Schritt ii) bei einer Temperatur im Bereich von 15-30 °C durchgeführt wird.

5. Verfahren nach einem der Ansprüche 1 bis 4, wobei das Verfahren die folgenden Schritte umfasst:
a) Bereitstellen von mindestens einer Art von DNA-Origami-Strukturen, die mindestens eine Art von Oligonukleotiden aufweist, die aus der DNA-Origami-Struktur herausragt und/oder in die DNA-Origami-Struktur eingebaut ist.
b) Bereitstellen von mindestens einer Art von Oligonukleotiden mit modifizierten Molekülen, wobei dieses Molekül, das durch π-Wechselwirkungen an Graphen binden kann, Pyren ist, und wobei das Oligonukleotid, das zu Purinen oder Pyrimidinen komplementäre Nukleobasen aufweist, zu den aus der DNA-Origami-Struktur herausragenden und/oder in die DNA-Origami-Struktur eingebauten Oligonukleotiden im Watson-Crickschen Sinne komplementär ist, wobei das komplementäre Oligonukleotid Nukleobasen enthält, die aus Purinen oder Pyrimidinen ausgewählt sind,
c) die DNA-Origami-Struktur mit dem Oligonukleotid mit modifizierten Molekülen wird länger als 5 min inkubiert, bevorzugt länger als 30 min, bevorzugter 30-120 min, bei einer Temperatur im Bereich von 10-40 °C, bevorzugt 30-38°C, um eine Watson-Crick-Paarung zwischen den Oligonukleotiden, die aus der DNA-Origami-Struktur herausragen und/oder in die DNA-Origami-Struktur eingebaut sind, und den komplementären Oligonukleotiden mit modifizierten Molekülen herzustellen.
d) die inkubierte DNA-Origami-Struktur wird in einer Temperatur im Bereich von 10-40°C länger als 10 Sekunden mit einer Graphenschicht kontaktiert, um die DNA-Origami-Strukturen an der Graphenschicht zu immobilisieren.

6. Verfahren nach Anspruch 5, wobei das Molekül, das durch π-Wechselwirkungen an Graphen binden kann, Pyren ist, und/oder die Anzahl der Pyren-Moleküle pro DNA-Origami-Struktur mindestens 2 und bevorzugt 4-10 ist.

7. Verfahren nach Anspruch 5 oder 6, wobei die Inkubationszeit im Schritt c) 2 Stunden bei 37°C beträgt.

8. Verfahren nach einem der Ansprüche 1 bis 7, wobei die DNA-Origami-Strukturen einen oder mehrere Arten von Objekten von Interesse (OOI) aufweisen, wobei die OOI ausgewählt sind aus der Gruppe bestehend aus Proteinen, Peptiden, Nanopartikeln, Kohlenhydraten, niedermolekularen Liganden, Farbstoffen, Nanokristallen, DNA, RNA und chemischen Gruppen wie Thiol oder Biotin.

9. Verfahren nach einem der Ansprüche 1 bis 8, wobei nach dem Schritt der Immobilisierung der DNA-Origami-Strukturen an der Graphenschicht ein weiterer Schritt durchgeführt wird, in dem die immobilisierte Probe mit einer beliebigen Lösung, in der DNA-Origami stabil ist, wie z. B. Reinstwasser, Phosphatgepufferte Salzlösung, 10 mM Tris oder 1xTris-EDTA-Puffer mit 1-15 mM MgCl2., gewaschen wird.

10. Durch Immobilisierung von DNA-Origami-Strukturen modifiziertes Graphen und Graphenschicht, wobei die DNA-Origami-Strukturen an der Graphenschicht durch Oligonukleotide mit modifizierten Molekülen immobilisiert sind, wobei dieses Molekül, das durch π-Wechselwirkungen an Graphen binden kann, Pyren ist, wobei dieses Molekül fähig ist, durch π-Wechselwirkungen an Graphen zu binden, und wobei diese Oligonukleotide mit modifizierten Molekülen, die zu Purinen oder Pyrimidinen komplementäre Nukleobasen aufweisen, für die Paarung mit den in den Nukleinsäurestrukturen der Oligonukleotiden DNA-Origami-Struktur enthaltenen Oligonukleotiden im Watson-Crickschen Sinne komplementär sind, wobei die komplementären Oligonukleotide Nukleobasen enthalten, die aus Purinen oder Pyrimidinen ausgewählt sind.

11. Durch Immobilisierung von DNA-Origami-Strukturen modifiziertes Graphen nach Anspruch 10, wobei die DNA-Origami-Struktur an der Graphenschicht durch Pyren-modifizierte Oligonukleotide immobilisiert ist, die zu Purinen oder Pyrimidinen komplementäre Nukleobasen enthalten, und für die Paarung mit den aus der DNA-Origami-Struktur herausragenden und/oder in die DNA-Origami-Struktur eingebauten Oligonukleotiden im Watson-Crickschen Sinne komplementär ist, wobei die komplementären Oligonukleotide Nukleobasen enthalten, die aus Purinen oder Pyrimidinen ausgewählt sind.

12. Durch Immobilisierung von DNA-Origami-Strukturen modifiziertes Graphen nach Anspruch 10 oder 11, wobei die DNA-Origami-Strukturen einen oder mehrere Arten von Objekten von Interesse (OOI) aufweisen, wobei die OOI ausgewählt sind aus der Gruppe bestehend aus Proteinen, Peptiden, Nanopartikeln, Kohlenhydraten, niedermolekularen Liganden, Farbstoffen, Nanokristallen, DNA, RNA und chemischen Gruppen wie Thiol oder Biotin.

13. Verwendung von durch Immobilisierung von DNA-Origami-Strukturen modifiziertem Graphen nach einem der Ansprüche 10 bis 12 in der Biosensorik und Energieumwandlung sowie in der Photovoltaik und Optoelektronik.

## Revendications

1. Procédé d'immobilisation de structures d'acide nucléique sur le graphène, ledit procédé comprenant les étapes suivantes :
i) fournir au moins un type de structures d'origami d'ADN comportant au moins un type d'oligonucléotide modifié par une molécule, ladite molécule étant capable de se fixer au graphène via des interactions π, ladite molécule capable de se fixer au graphène via des interactions π étant le pyrène,
ii) les structures d'origami d'ADN comportant au moins un type d'oligonucléotide modifié par la molécule sont mises en contact avec une couche de graphène à une température comprise dans la plage de 10-40°C, afin d'immobiliser les structures d'origami d'ADN sur le graphène.

2. Procédé selon la revendication 1, dans lequel l'étape i) comprend les sous-étapes suivantes :
- fournir au moins un type de structure d'origami d'ADN comportant au moins un type d'oligonucléotide,
- fournir au moins un type d'oligonucléotide modifié par une molécule, ladite molécule étant capable de se fixer au graphène via des interactions π, ladite molécule capable de se fixer au graphène via des interactions π étant le pyrène, et l'oligonucléotide modifié par la molécule, comprenant des bases nucléiques complémentaires aux purines ou aux pyrimidines, étant complémentaire au sens de Watson-Crick à au moins un type d'oligonucléotide compris dans la structure d'origami d'ADN, l'oligonucléotide complémentaire comprenant des bases nucléiques sélectionnées parmi les purines ou les pyrimidines,
- la structure d'origami d'ADN avec l'oligonucléotide modifié par la molécule est incubée pendant plus de 5 min, de préférence pendant plus de 30 min, de manière plus préférée pendant 30-120 min, à une température comprise dans la plage de 10-40°C, de manière plus préférée de 30-38°C, afin d'apparier selon la manière de Watson-Crick les oligonucléotides des structures d'origami d'ADN et l'oligonucléotide complémentaire modifié par la molécule.

3. Procédé selon la revendication 1 ou 2, dans lequel l'étape i) comprend la sous-étape suivante consistant à :
- former des structures d'origami d'ADN en utilisant des brins agrafes modifiés par une molécule, ladite molécule étant capable de se fixer au graphène via des interactions π.

4. Procédé selon l'une quelconque des revendications 1 à 3, dans lequel l'étape ii) est réalisée pendant au moins 10 secondes, et/ou l'étape ii) est réalisée à une température comprise dans la plage de 15-30°C.

5. Procédé selon l'une quelconque des revendications 1 à 4, ledit procédé comprenant les étapes suivantes :
a) fournir au moins un type de structure d'origami d'ADN comportant au moins un type d'oligonucléotide faisant saillie de la structure d'origami d'ADN et/ou intégré dans la structure d'origami d'ADN,
b) fournir au moins un type d'oligonucléotide modifié par une molécule, ladite molécule capable de se fixer au graphène via des interactions π étant le pyrène, et ledit oligonucléotide comprenant des bases nucléiques complémentaires aux purines ou aux pyrimidines étant complémentaire au sens de Watson-Crick aux oligonucléotides faisant saillie de la structure d'origami d'ADN et/ou intégrés dans la structure d'origami d'ADN, l'oligonucléotide complémentaire comprenant des bases nucléiques choisies parmi les purines ou les pyrimidines,
c) la structure d'origami d'ADN avec l'oligonucléotide modifié par la molécule est incubée pendant plus de 5 min, de préférence plus de 30 min, de manière plus préférée pendant 30 à 120 min, à une température comprise dans la plage de 10-40°C, de manière plus préférée de 30-38°C, afin d'apparier selon la manière de Watson-Crick les oligonucléotides faisant saillie de la structure d'origami d'ADN et/ou intégrés dans la structure d'origami d'ADN et les oligonucléotides complémentaires modifiés par la molécule,
d) la structure d'origami d'ADN incubée est mise en contact avec une couche de graphène pendant plus de 10 secondes, à une température comprise dans la plage de 10-40°C, afin d'immobiliser les structures d'origami d'ADN sur la couche de graphène.

6. Procédé selon la revendication 5, dans lequel ladite molécule capable de se fixer au graphène via des interactions π est le pyrène, et/ou le nombre de molécules de pyrène par structure d'origami d'ADN est d'au moins 2, de préférence de 4 à 10.

7. Procédé selon la revendication 5 ou 6, dans lequel l'incubation de l'étape c) est réalisée pendant 2 heures à 37°C.

8. Procédé selon l'une quelconque des revendications 1 à 7, dans lequel lesdites structures d'origami d'ADN portent un ou plusieurs types d'objets d'intérêt (OOI), les OOI étant choisis dans le groupe comprenant des protéines, des peptides, des nanoparticules, des glucides, des ligands de type petite molécule, des colorants, des nanocristaux, de l'ADN, de l'ARN, des groupements chimiques tels que le thiol ou la biotine.

9. Procédé selon l'une quelconque des revendications 1 à 8, dans lequel, après l'étape d'immobilisation de la structure d'origami d'ADN sur la couche de graphène, est réalisée une étape supplémentaire de lavage de l'échantillon immobilisé avec toute solution dans laquelle l'origami d'ADN est stable, telle que de l'eau ultrapure, un tampon salin phosphate (PBS), un tampon Tris 10 mM ou un tampon Tris-EDTA 1x contenant 1 à 15 mM de MgCl₂.

10. Graphène modifié par l'immobilisation de structures d'origami d'ADN et d'une couche de graphène, les structures d'origami d'ADN étant immobilisées sur la couche de graphène via des oligonucléotides modifiés par une molécule, ladite molécule, qui est capable de se fixer au graphène via des interactions π, étant le pyrène, ladite molécule étant capable de se fixer au graphène via des interactions π, et lesdits oligonucléotides modifiés par une molécule, comprenant des bases nucléiques complémentaires aux purines ou aux pyrimidines, étant complémentaires au sens de Watson-Crick pour l'appariement aux oligonucléotides compris dans lesdites structures d'acide nucléique, les oligonucléotides complémentaires comprenant des bases nucléiques choisies parmi les purines ou les pyrimidines.

11. Graphène modifié par l'immobilisation de structures d'origami d'ADN selon la revendication 10, dans lequel ladite structure d'origami d'ADN est immobilisée sur la couche de graphène via des oligonucléotides modifiés par du pyrène, comprenant des bases nucléiques complémentaires aux purines ou aux pyrimidines, complémentaires au sens de Watson-Crick pour l'appariement aux oligonucléotides faisant saillie de la structure d'origami d'ADN et/ou intégrés dans ladite structure d'origami d'ADN, les oligonucléotides complémentaires comprenant des bases nucléiques choisies parmi les purines ou les pyrimidines.

12. Graphène modifié par l'immobilisation de structures d'origami d'ADN selon la revendication 10 ou 11, lesdites structures d'origami d'ADN portant un ou plusieurs types d'objets d'intérêt (OOI), les OOI étant choisis dans le groupe comprenant des protéines, des peptides, des nanoparticules, des glucides, des ligands de type petite molécule, des colorants, des nanocristaux, de l'ADN, de l'ARN, des groupements chimiques tels que le thiol ou la biotine.

13. Utilisation du graphène modifié par l'immobilisation de structures d'origami d'ADN tel que défini dans l'une quelconque des revendications 10 à 12 dans la biodétection et la conversion d'énergie, ainsi que dans le photovoltaïque et l'optoélectronique.
